# EUROPÄISCHE PATENTSCHRIFT

(11) **EP 4 313 301 B1**
(45) Veröffentlichungstag und Bekanntmachung des Hinweises auf die Patenterteilung: **25.02.2026**
(21) Anmeldenummer: 22719808.2
(22) Anmeldetag: 25.03.2022
(51) Int. Cl.: C07K 14/47, A61P 25/28, A61K 38/00, C07K 19/00

(54) **VERWENDUNG VON D-ENANTIOMEREN PEPTIDIDLIGANDEN VON MONOMEREM TAU FÜR DIE THERAPIE VERSCHIEDENER TAUOPATHIEN**
USE OF D-ENANTIOMERIC PEPTIDE LIGANDS OF MONOMERIC TAU FOR THE THERAPY OF VARIOUS TAUOPATHIES
UTILISATION DE LIGANDS PEPTIDIQUES D-ÉNANTIOMÈRES DE PROTÉINE TAU MONOMÈRE POUR LA THÉRAPIE DE DIVERSES TAUOPATHIES

(30) Priorität: 25.03.2021 DE 102021107546
(43) Veröffentlichungstag der Anmeldung: 07.02.2024
(73) Patentinhaber: Priavoid GmbH, 40225 Düsseldorf (DE)
(72) Erfinder: WILLBOLD, Dieter, 52428 Jülich (DE); MOHRLÜDER, Jeannine, 41812 Erkelenz (DE); SEVENICH, Marc, 53909 Zülpich (DE); SANTUR, Karoline Bianka, 51143 Köln (DE); ALTENDORF, Tim, 52146 Würselen (DE)
(74) Vertreter: Grünecker Patent- und Rechtsanwälte PartG mbB
(86) Internationale Anmeldenummer: PCT/EP2022/057923
(87) Internationale Veröffentlichungsnummer: WO 2022/200572

(56) Entgegenhaltungen:
- WO-A1-2021/023719
- DE-A1- 102012 019 882
- ZHANG XIANCHENG ET AL: "Selection of a D-Enantiomeric Peptide Specifically Binding to PHF6 for Inhibiting Tau Aggregation in Transgenic Mice", ACS CHEMICAL NEUROSCIENCE, vol. 11, no. 24, 7 December 2020 (2020-12-07), US, pages 4240 - 4253, XP055935512, ISSN: 1948-7193, DOI: 10.1021/acschemneuro.0c00518
- ZHANG XIANCHENG ET AL: "Supporting Information - Selection of a D-Enantiomeric Peptide Specifically Binding to PHF6 for Inhibiting Tau Aggregation in Transgenic Mice", ACS CHEMICAL NEUROSCIENCE, vol. 11, no. 24, 16 December 2020 (2020-12-16), US, pages 4240 - 4253, XP055935510, ISSN: 1948-7193, DOI: 10.1021/acschemneuro.0c00518
- CHRISTINA DAMMERS ET AL: "Potent Tau Aggregation Inhibitor D-Peptides Selected against Tau-Repeat 2 Using Mirror Image Phage Display", PLOS ONE, vol. 11, no. 12, 22 December 2016 (2016-12-22), pages e0167432, XP055646356, DOI: 10.1371/journal.pone.0167432
- MALHIS MARWA ET AL: "Potent Tau Aggregation Inhibitor D-Peptides Selected against Tau-Repeat 2 Using Mirror Image Phage Display", CHEMBIOCHEM, vol. 22, no. 21, 10 August 2021 (2021-08-10), pages 3049 - 3059, XP055935508, ISSN: 1439-4227, Retrieved from the Internet <URL:https://onlinelibrary.wiley.com/doi/full-xml/10.1002/cbic.202100287> DOI: 10.1002/cbic.202100287

## Beschreibung

Die Erfindung betrifft ein Peptid, bestehend aus einer Aminosäuresequenz ausgewählt aus der Gruppe bestehend aus SEQ ID NO: 1, SEQ ID NO: und SEQ ID NO: 5 sowie ein solches Peptid zur Verwendung in der Behandlung von Tauopathien.

Die Alzheimer'sche Demenz (AD) ist einem geschätzten Anteil von knapp zwei Dritteln - was ca. 27 Millionen Menschen entspricht - die häufigste neurodegenerative Erkrankung weltweit. Sie wird einer heterogenen Gruppe verschiedener Tauopathien zugeordnet, welche symptomatisch durch eine fortschreitende Demenz gekennzeichnet sind. Die dabei im Gehirn auftretenden intraneuronalen Ablagerungen, welche amyloide Strukturen des hyperphosphorylierten Tau Proteins beinhalten, korrelieren meist mit dem Ausmaß kognitiver Defizite im Krankheitsverlauf.

Tau ist als neuronales Mikrotubuli-assoziiertes Protein maßgeblich in die Assemblierung und Stabilisierung von Mikrotubuli involviert.

Hyperphosphorylierung von Tau triggert dessen Fehlfaltung und Aggregation bis hin zur Ausbildung von sog. neurofibrillären Bündeln, welche ein neuropathologisches Kennzeichen der meisten Tauopathien sind. Im Gehirn erwachsener Menschen werden durch alternative Splicing sechs Tau Isoformen exprimiert, welche zwischen 352 und 441 Aminosäuren lang sind. Abhängig von der Zusammensetzung an vorliegenden Isoformen können unterschiedliche Fehlkonformationen ausgebildet werden, welche spezifisch für die vorliegende Tau-Pathologie ist.

Toxische, selbst-replizierende und propagierende Tau-Aggregate sind ein Kennzeichen verschiedener Tauopathien. Bei diesen Pathologien liegt eine charakteristische Prion-ähnliche Verbreitung von fehlgefalteten Tau-Aggregaten im ZNS vor, die mit einer Neurodegeneration der betroffenen Hirnareale einhergeht. Mehrere Studien konnten die Aufnahme von Tau-Aggregaten von Zellen, "Seeding" Effekte (Aggregate, welche als Templat die Umwandlung von monomerem zu fehlgefaltetem, oligomerem oder fibrillärem Tau induzieren), sowie Tau-Aggregattransfer zwischen Zellkulturen nachweisen. Die Verbreitungswege der Tau-assoziierten Pathologie reflektieren hierbei neuroanatomisch verbundene Hirnregionen.

Bisher existiert kein Wirkstoff oder Medikament, das gegen die Ursachen von AD wirkt. Die bisher eingesetzten und zugelassenen Medikamente mildern einige der bei der Alzheimerschen Demenz auftretenden Symptome. Sie sind aber nicht in der Lage, den Krankheitsfortschritt zu verlangsamen oder eine Heilung herbeizuführen. Es existieren einige Substanzen, die im Tierversuch Erfolge bei der Prävention, aber nicht (unbedingt) bei der Behandlung von AD gezeigt haben.

Allerdings sind fünf Medikamente (Aricept, Exelon, Razadyne, Donepezil und Namenda) erhältlich, welche die Symptome der Krankheit lindern und den Betroffenen den Alltag erleichtern.

Die Frontotemporale Demenz ist ebenfalls nicht ursächlich behandelbar, so daß auch hier der Krankheitsverlauf weder verlangsamt noch gestoppt werden kann. In erster Linie werden Antidepressiva eingesetzt, welche symptomlindernd wirken können.

Für die Progressive supranukleäre Blickparese ist bisher keine Möglichkeit vorhanden, die Pathologie aufzuhalten oder zu verlangsamen. Einige Symptome sprechen auf keinerlei Medikation an. Antidepressiva (z.B. Prozac, Elavil und Tofranil) werden zur Linderung weiterer Symptome eingesetzt, Spezialbrillen und Gehhilfen dienen der Erleichterung des Alltags.

Die durch eine Maserninfektion ausgelöste subakute sklerosierende Panenzephalitis kann zum heutigen Zeitpunkt nicht geheilt werden. Antivirale Therapeutika (Isoprinosine und Ribavirin), sowie immunmodulierende Substanzen (Interferon Alpha) können den Krankheitsfortschritt stoppen und die Lebenserwartung der Patienten erhöhen. Allerdings sind die Nebenwirkungen einer Langzeitbehandlung bisher unbekannt.

Bisher ist für die Corticobasale Degeneration kein Medikament vorhanden, welches den Krankheitsverlauf verlangsamt oder aufhält. Die Symptome der Corticobasalen Degeneration sind meist therapieresistent. Die Substanz Clonazepam wird gegen Myoklonie eingesetzt. Physio- und Sprachtherapien können bei der Alltagsbewältigung helfen.

ZHANG XIANCHENG ET AL., ACS CHEMICAL NEUROSCIENCE, Bd. 11, Nr. 24, 7. Dezember 2020 Seiten 4240-4253, beschreiben D-Peptide, die gegen Tau gerichtet sind und für die Therapie von Tauopathien verwendet werden können.

Zusammenfassend ist eine ursächliche und deutlich lebensverlängernde Therapie ist für die meisten, wenn nicht allen, Tauopathien bisher nicht vorhanden und wird dringend benötigt.

Die Aufgabe der vorliegenden Erfindung war daher die Entwicklung von neuen chemischen Einheiten, welche die Bildung neuer Aggregate minimieren, sowie bereits existierende, toxische Tau-Aggregate in native funktionelle Tau-Monomere disassemblieren können und so ihr therapeutischer Einsatz bei unterschiedlichen Tauopathien möglich ist.

Die in der Therapie einzusetzende chemisch Einheit bzw. seine Varianten sollen möglichst affin und spezifisch an das native, endogene, monomere Tau-Protein binden und es damit stabilisieren. Das Gleichgewicht aus fehlgefalteter und nativ gefalteter Tau-Konformation wird somit zugunsten der letzteren verschoben. So können im Idealfall bereits schon bestehende Tau-Oligomere und Fibrillen in ihre Monomerbausteine zerlegt und damit eliminiert werden.

Diese Aufgabe wurde durch ein Peptid gemäß Anspruch 1 1

Weitere bevorzugte Ausführungsformen sind in den abhängigen Ansprüchen definiert.

Im Folgenden soll der Begriff "umfassen" auch "bestehen aus" beinhalten.

Die Peptide der SEQ ID NO: 1, SEQ ID NO: 2, SEQ ID NO: 3, SEQ ID NO: 4, SEQ

ID NO: 5 SEQ ID NO: 6 oder SEQ ID NO: 7 wurden mit Hilfe einer optimierten Spiegelbild-Phagendisplay-Selektion gefunden. Die Peptide der SEQ ID NO: 3, SEQ ID NO: 4, SEQ ID NO: 6 und SEQ ID NO: 7 sind nicht beansprucht.

Es sei angemerkt, dass das Verfahren zum Spiegelbildphagendisplay neben der hier angegebenen Selektion von spezifisch Monomerbindern selbstverständlich auch zum Auffinden von spezifisch Oligomerbindern oder sogar zum Auffinden von Liganden gegenüber anderen bei Proteinfehlfaltungserkrankung auftretenden Spezies genutzt werden kann.

Im Spiegelbild-Phagendisplay wird z. B. eine rekombinante Bibliothek von randomisierten Peptidsequenzen, präsentiert am gp3 Protein des M13-Phagen und codiert in dessen Genom, gegen das genaue Spiegelbild (D-enantiomer) eines natürlich vorkommenden L-enantiomeren Zielmoleküls (z. B. Tau) selektiert.

Die Peptidsequenz wird vorteilhaft am N-Terminus des gp3-Proteins des M13-Phagen präsentiert und liegt codiert in dessen Genom vor.

Das gp3-Molekül auch gene product 3 genannt, ist ein Protein, welches in der Hülle des Phagen sitzt und für den Kontakt mit der Wirtszelle benötigt wird.

Die DNA-Sequenz des p3-Gens eines selektierten Phagen ist verknüpft mit der DNA-Sequenz, die die genetische Information über die korrespondierende Peptidsequenz am gp3-Molekül enthält, wodurch diese sequenziert werden kann. Nach der Sequenzierung kann die genomische Sequenz in eine Aminosäuresequenz umgeschrieben werden und als D-enantiomeres Peptid, welches an die physiologische L-enantiomere Form des Zielmoleküls (z. B. Tau) bindet, synthetisiert werden. Die Gesamtheit der Phagen, welche unterschiedliche Peptide als Fusionsprotein mit gp3 auf ihrer Oberfläche präsentieren wird im Folgenden als Phagenbibliothek bezeichnet. Die entsprechenden Peptide stellen die im Versuch zu selektierenden Biomoleküle dar.

Es können sogenannte panning-Runden durchgeführt werden, z. B. drei Runden. Dabei wird die Phagenbibliothek mit einem fixierten Zielmolekül, auch Köder genannt, in Kontakt gebracht und bindende Phagen aus dem milliardenfachen Hintergrund anderer, nicht-bindender Phagen isoliert.

Beispielhaft wird die Menge an Phagen reduziert, die bevorzugt an oligomere oder fibrilläre Spezies von Tau binden, indem eben diese Spezies nicht als Köder angeboten werden. Phagen, die eine erhöhte Affinität zu Tau-Oligomeren und - Fibrillen zeigen, können auf diese Weise aus dem Phagenpool entfernt werden, so dass sich z. B. Tau-Monomer spezifische Phagen anreichern. Das Verfahren kann selbstverständlich in analoger Weise angepasst werden, um spezifisch Tau-Oligomer bindende Liganden und Peptide zu ermitteln.

Um eine Anreicherung von Plastik-, BSA- oder Streptavidin-affinen Phagen zu verringern, werden erfindungsgemäß zudem in vorzugsweise allen panning-Runden verschiedene Substratoberflächen verwendet. Als Substratoberfläche ist in diesem Fall eine Kombination aus dem verwendeten Substrat (Plastikplatte, in diesem Fall Polystyrol mit einer Streptavidin Matrix )und den verwendeten Blockierungs- bzw. Quenchingagenzien definiert. In den aufeinanderfolgenden Selektionsrunden wird sukzessive der Selektionsdruck erhöht.

Dazu wird, während die Konzentration des Zielmoleküls (z. B. monomeres Tau) stabil bleibt, ab der 2. Selektionsrunde kontinuierlich die Waschschrittanzahl nach der Phageninkubation erhöht, um nicht Tau-Monomer affine Phagen zu entfernen.

Des Weiteren wird in jeder Selektionsrunde des Phagendisplay eine andere Substratoberfläche gewählt, indem nach der Immobilisierung des Zielmoleküls auf dem Substrat andere Agenzien zum Blockieren der Oberfläche genutzt werden (z.B. BSA, Milchpulver, keine Blockierung). Beispielhaft kann zwischen einer zusätzlich zur Biotin-behandelten Milchpulver-blockierten Oberfläche in Runde 1, einer BSA-blockierten-Oberfläche in Runde 2 und einer Milchpulver behandelten-Oberfläche in Runde 3 gewechselt werden.

Der Wechsel zwischen verschiedenen Substratoberflächen steigert die Spezifität für das Zielmolekül bzw. den Köder, gegenüber der Oberfläche. Außerdem erfolgt eine Verringerung der Liganden, die unspezifisch an Plastikoberflächen oder das Blockingagens binden.

Parallel zur eigentlichen Phagendisplayselektion können beispielhaft Kontrollselektionen durchgeführt werden, welche in Bezug auf die Durchführung mit der Hauptselektion identisch sind - mit dem wichtigen Unterschied, dass hier kein Köder eingesetzt wird. Eine Datenanalyse der Sequenzen, welche aus den Kontrollselektionen resultieren, ermöglicht die Identifizierung von Peptiden, welche sich auch ohne Köder während der Selektion anreichern und somit für alle folgenden Schritte irrelevant sind.

Das Verfahren ist somit gekennzeichnet durch folgende Schritte:
a) Bereitstellen eines immobilisierten Köders auf einer Substratoberfläche.
b) In Kontakt bringen des als Köder fungierenden immobilisierten Moleküls mit einer Lösung, die eine Bibliothek von Molekülen enthält.
c) In Kontakt bringen der mit den Molekülen besetzten immobilisierten Köder mit einer Waschlösung.
d) Trennung und Vermehrung der nach dem in Kontakt bringen der mit den Molekülen besetzten immobilisierten Köder mit Waschlösung weiterhin an den Köder gebundenen Moleküle.
e) Wiederholung der genannten Schritte, wobei bei jeder Wiederholung ein unterschiedliches Substrat verwendet wird,
f) Identifizierung der Sequenz der nach der Wiederholung auf den Köder verbleibenden Moleküle.

Ein unterschiedliches Substrat wird z. B. durch den Wechsel der Substratart und/oder dessen Blockierung oder Nichtblockierung mittels Reagenzien genutzt.

Als Köder wird ein Molekül aus der Gruppe bestehend aus Proteinen, Peptiden, RNA, DNA, m-RNA und chemischen Verbindungen eingesetzt. Als Köder wird im vorliegenden Fall insbesondere monomeres Tau Protein verwendet.

Als Oberfläche bzw. Substrat, an die der Köder immobilisiert wird, wird z. B. eine Komponente aus der Gruppe bestehend aus Mikrotiterplatten, Magnetpartikel, Agarose- oder Sepharosekügelchen eingesetzt.

Bei dem Köder nach Punkt a) handelt es sich also um eine Verbindung, an die das zu selektierende Biomolekül gebunden werden soll. Er wird nach dem Stand der Technik bekannten Methoden an eine erste Oberfläche fixiert. Beispielhaft aber nicht beschränkend können als Köder Proteine, Peptide, RNA oder DNA-Moleküle genannt werden, insbesondere Tau-Monomer. Als mögliche Oberflächen können beispielhaft Mikrotiterplatten, Magnetpartikel, Agarose- oder Sepharosekügelchen verwendet werden.

Die Oberfläche mit dem immobilisierten Köder kann anschließend gequencht werden, wobei die funktionellen Gruppen des Substrates inaktiviert werden. Außerdem kann eine Blockierung der auf dem Substrat verbleibenden freien Flächen mit geeigneten Reagenzien erfolgen.

Im zweiten Schritt b) wird der immobilisierte Köder mit einer randomisierten Bibliothek von Molekülen - speziell Biomolekülen - in Kontakt gebracht. Diese Biomoleküle konkurrieren um die Bindung an den Köder. Bei der randomisierten Bibliothek handelt es sich um eine Mischung von sehr vielen, beispielsweise 10¹², aber auch 10⁴ oder nur 100 verschiedenen Molekülen in einer Mischung. Eine solche Bibliothek kann beispielsweise jeweils aus Peptiden, Proteinen, DNA, RNA oder m-RNA bestehen, welche an bestimmten Vehikeln gebunden vorliegen, und die an Köder anbinden können. Als Vehikel kommen beispielsweise Phagen, Polysomen oder Bakterienoberflächen in Betracht. Die Bibliothek kann aus artifiziellen oder aus der Natur isolierten Bestandteilen oder aus einer Mischung von beidem bestehen. Unter artifiziell im Sinne der Erfindung sind beispielsweise aus der Oligonukleotidsynthese hergestellte Verbindungen zu verstehen.

Es kann der mit Biomolekülen besetzte immobilisierte Köder in Schritt c) mit einer Waschsubstanz in Kontakt gebracht werden. Hierzu wird in Schritt c) ein Waschschritt durchgeführt, bei dem eine Pufferlösung mit den immobilisierten Ködern in Kontakt gebracht bzw. gespült wird. Dies bedeutet, dass die Lösung mit der Bibliothek von Biomolekülen vorzugsweise wiederholt durch eine ggf. ähnliche oder identische Lösung ausgetauscht wird. Somit werden diejenigen Bibliotheksmoleküle entfernt, welche weniger schnell von den immobilisierten Ködern abdissoziieren als andere Bibliotheksmoleküle. Die Schnelligkeit der Ablösungsreaktion der bindenden Bibliotheksmoleküle wird dabei hauptsächlich durch die unterschiedlichen Dissoziationskonstanten (insbesondere die k_{off}-Werte) der einzelnen Moleküle bestimmt. Solche mit einem kleinen k_{off}-Wert bleiben statistisch gesehen am längsten am immobilisierten Köder gebunden und haben damit eine geringere statistische Wahrscheinlichkeit, durch den Waschpuffer weggewaschen zu werden. Die den Waschpuffer enthaltene Flüssigkeit ist vorzugsweise wässrig und kann einen pH-Puffer enthalten. Fakultative Komponenten der Lösung für den Waschschritt können Salze, Detergentien oder Reduktionsmittel sein.

Nach dem Spezifitätswaschschritt in Schritt c) werden in Schritt d) die gebundenen Biomoleküle vom Köder getrennt und vermehrt.

Die Trennung in Schritt d) erfolgt z. B. durch Elution von Phagen vom Köder. Die Trennung kann beispielsweise durch Änderung des pH-Wertes, Erhitzen oder Änderung, insbesondere Erhöhung der Salzkonzentration erfolgen. Bei der nachfolgenden Vermehrung der noch am Köder verbliebenen Bibliotheksmoleküle können die beispielsweise nach den Schritten a) bis c) gewonnene Phagenpartikel in Zellen eingebracht und vermehrt werden.

Bei Schritt e) ist die Konzentration der selektierten Biomoleküle in der Lösung, die dem Köder nach Schritt a) zugeführt wird, erhöht. Vorzugsweise werden 3 bis 6 Selektionsrunden, die die Schritte a) bis e) enthalten, durchgeführt. Es können aber auch 1 bis 10 oder 1 bis 20 Wiederholungen durchgeführt werden. Auch die dabei vorzugsweise vorgenommene Erhöhung der Kompetitorenkonzentration in Schritt c) bei zunehmender Zyklenzahl führt zu einer verbesserten Selektion.

Ein besonders relevantes Spiegelbildphagendisplay sieht N-terminal biotinyliertes D-enantiomeres Tau-Monomer in Schritt a) vor, eine rekombinante Phagenbibliothek in Schritt b) sowie eine Pufferlösung in Schritt c) neben Tau-Monomer als Köder. Eine Eluierung als Trennschritt erfolgt z. B. über pH-Wert-Erniedrigung als Trennschritt und Phagen-Amplifikation in Bakterienzellen als Vermehrung.

Auf diese Weise wurden sieben D-enantiomere Peptide, die spezifisch gegen Tau-Monomer binden, selektiert.
SEQ ID NO 1:
   TF2D-1 (freier N-terminus, amidierter C-terminus):
   nemylwhwqypqhlrvg
SEQ ID NO 2:
   TF2D-5 (freier N-terminus, amidierter C-terminus):
   dggyqilfkipgghih
SEQ ID NO 3:
   TF2D-1a (freier N-terminus, amidierter C-terminus):
   nemylwhwqypqhlrvrrrrr
SEQ ID NO 4:
   TF2D-1b (freier N-terminus; amidierter C-terminus):
   nelylwhwqypqhlrvrrrrr
SEQ ID NO 5:
   TF2D-5a (freier N-terminus, amidierter C-terminus):
   dggyqilfkipgghihrrrrr
SEQ ID NO 6:
   TF2D-5b (freier N-terminus, amidierter C-terminus):
   rssyqilfripsshihrrrrr
SEQ ID NO 7:
   TF2D-5c (freier N-terminus, amidierter C-terminus):
   yqilfripsshihrrrrr

Die Peptide gemäß der SEQ ID NO: 1 bis 7 können durch die spezifische Bindung an Tau-Monomere, als mögliches Medikament gegen Tauopathien, wie die Alzheimersche Demenz genutzt werden.

Unter Tau-Peptid bzw. Tau-Protein wird hier bevorzugt das humane Tau-Peptid bzw. Tau-Protein verstanden.

Die erfindungsgemäßen Peptide sind ferner vorzugsweise dadurch gekennzeichnet, dass am freien C-terminus an Stelle der Carboxylgruppe eine Säureamidgruppe (CONH₂-Gruppe) oder eine COH-Gruppe, COCI-Gruppe, COBr-Gruppe, CONH-alkyl-Rest oder ein CONH-alkyl-Amin-Rest vorliegt oder aber das Peptid zyklisiert vorliegt.

Damit wird besonders vorteilhaft zusätzlich die Aufgabe gelöst, dass ein Peptid ohne negative Ladung am C-terminus, bereitgestellt wird. Dadurch wird vorteilhaft bewirkt, dass dieses mit höherer Affinität an das Zielmolekül binden kann, als ein Peptid, welches eine Carboxylgruppe am freien C-terminus aufweist. Peptide mit freier, nichtmodifizierter Carboxylgruppe weisen im physiologischen Zustand eine negative Ladung an diesem Ende auf.

In einer Ausgestaltung der Erfindung sind die erfindungsgemäßen Peptide im physiologischen Zustand, insbesondere bei pH 6-8, insbesondere 6,5-7,5, insbesondere bei pH 6,0, pH 6,1, pH 6,2, pH 6,3, pH 6,4, pH 6,5 pH 6,6, pH 6,7, pH 6,8, pH 6,9, pH 7,0, pH 7,1, pH 7,2, pH 7,3, pH 7,4, pH 7,5, pH 7,6, pH 7,7, pH 7,8, pH 7,9 bzw. pH 8,0 derart modifiziert, dass der C-terminus keine negative Ladung trägt, sondern stattdessen neutral ist oder eine oder mehrere positive Ladungen aufweist.

In einer Ausführungsform ist das Peptid dadurch gekennzeichnet, dass am freien C-terminus an Stelle der Carboxylgruppe eine Säureamidgruppe vorliegt. Statt der Carboxylgruppe (-COOH-Gruppe) ist also eine Säureamidgruppe (-CONH₂-Gruppe) am C-terminus angeordnet.

Das Peptid ist demnach besonders vorteilhaft am freien C-Terminus amidiert und am freien N-Terminus nicht modifiziert.

Das Peptid ist demnach besonders vorteilhaft am freien C-Terminus amidiert und am freien N-Terminus nicht modifiziert.

Dadurch wird besonders vorteilhaft die weitere Aufgabe gelöst, dass ein Peptid ohne negativen Ladungsüberschuss vorliegt, welches affiner an das Zielmolekül binden kann und auf einfache Weise erhältlich ist.

In einer weiteren Ausgestaltung der Erfindung liegen folgende weitere Gruppen an Stelle der Carboxylgruppe vor: COH, COCl, COBr, CONH-alkyl-Rest, CONH-alkyl-Amin-Rest (positive Netto-Ladung) etc., wobei man hierauf nicht beschränkt, sofern der technischen Lehre des Hauptanspruchs gefolgt wird.

In einer weiteren bevorzugten Ausgestaltung der Erfindung ist daher die Affinität der Bindung der erfindungsgemäß modifizierten Peptide ohne negative Ladung am C-terminus im Vergleich zu linearen Peptiden mit negativer Ladung am C-terminus aber im Übrigen gleicher Aminosäuresequenz, um 1%, 2, 3, 4, 5, 6, 7, 8, 9, insbesondere 10%, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, insbesondere 100%, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, insbesondere 200%, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, insbesondere 300%, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, insbesondere 400%, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, vorteilhaft sogar 500%, 501, 502, 503, 504, 505, 506, 507, 508, 509, 510, 511, 512, 513, 514, 515, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, besonders vorteilhaft 600%, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 698, 699, besonders vorteilhaft 700%, 701, 702, 703, 704, 705, 706, 707, 708, 709, 710, 711, 712, 713, 714, 715, 716, 717, 718, 719, 720, 721, 722, 723, 724, 725, 726, 727, 728, 729, 730, 731, 732, 733, 734, 735, 736, 737, 738, 739, 740, 741, 742, 743, 744, 745, 746, 747, 748, 749, 750, 751, 752, 753, 754, 755, 756, 757, 758, 759, 760, 761, 762, 763, 764, 765, 766, 767, 768, 769, 770, 771, 772, 773, 774, 775, 776, 777, 778, 779, 780, 781, 782, 783, 784, 785, 786, 787, 788, 789, 790, 791, 792, 793, 794, 795, 796, 797, 798, 799, ebenfalls besonders vorteilhaft 800%, 801, 802, 803, 804, 805, 806, 807, 808, 809, 810, 811, 812, 813, 814, 815, 816, 817, 818, 819, 820, 821, 822, 823, 824, 825, 826, 827, 828, 829, 830, 831, 832, 833, 834, 835, 836, 837, 838, 839, 840, 841, 842, 843, 844, 845, 846, 847, 848, 849, 850, 851, 852, 853, 854, 855, 856, 857, 858, 859, 860, 861, 862, 863, 864, 865, 866, 867, 868, 869, 870, 871, 872, 873, 874, 875, 876, 877, 878, 879, 880, 881, 882, 883, 884, 885, 886, 887, 888, 889, 890, 891, 892, 893, 894, 895, 896, 897, 898, 899, ebenfalls besonders vorteilhaft 900%, 901, 902, 903, 904, 905, 906, 907, 908, 909, 910, 911, 912, 913, 914, 915, 916, 917, 918, 919, 920, 921, 922, 923, 924, 925, 926, 927, 928, 929, 930, 931, 932, 933, 934, 935, 936, 937, 938, 939, 940, 941, 942, 943, 944, 945, 946, 947, 948, 949, 950, 951, 952, 953, 954, 955, 956, 957, 958, 959, 960, 961, 962, 963, 964, 965, 966, 967, 968, 969, 970, 971, 972, 973, 974, 975, 976, 977, 978, 979, 980, 981, 982, 983, 984, 985, 986, 987, 988, 989, 990, 991, 992, 993, 994, 995, 996, 997, 998, 999, oder sogar um 1000%, oder sogar um 10000% oder sogar um bis zu 100000% oder 1000000% erhöht, wobei jeder Zwischenwert angenommen werden kann.

Dies wird durch einen entsprechend erniedrigten K_{D}-Wert angezeigt. Der K_{D}-Wert als Maß für die Affinität der Bindung eines modifizierten Peptids an Tau-Monomer ist im Vergleich zu einem linearen, bindenden Peptid mit negativer Ladung am freien C-terminus, um 1%, 2, 3, 4, 5, 6, 7, 8, 9, insbesondere 10%, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, insbesondere 99,1, 99,2, 99,3, 99,4, 99,5%, 99,6, 99,7, 99,8, 99,9 bis zu 99,99 oder sogar 99,999% erniedrigt, wobei jeder Zwischenwert angenommen werden kann.

Das erfindungsgemäße Peptid ist ferner vorzugsweise dadurch gekennzeichnet, dass es 2, 3, 4, 5, 6, 7, 8, 9, 10 oder mehr Kopien der Sequenzen mit der SEQ ID NO: 1, SEQ ID NO: 2, und/oder SEQ ID NO: 5 enthält.

Es sind auch Varianten denkbar, wobei das Peptid 11, 12, 13, 14, 15, 16, 17, 18, 19, 20 oder mehr Peptide mit der SEQ ID NO: 1, SEQ ID NO: 2, und/oder SEQ ID NO: 5 enthält.

Insbesondere bevorzugt sind Dimere der Sequenzen mit der SEQ ID NO: 1, SEQ ID NO: 2, und/oder SEQ ID NO: 5, wobei es sich bei den beiden Monomeren des Dimers um Peptide mit der gleichen SEQ ID oder mit einer verschiedenen SEQ ID handelt.

Die erfindungsgemäßen Peptide sind dadurch gekennzeichnet, dass sie aus D-Aminosäuren bestehen.

Das erfindungsgemäße Peptid ist ferner vorzugsweise dadurch gekennzeichnet, dass das Peptid mit einer weiteren Substanz verknüpft ist.

Bei der Verknüpfung handelt es sich im Sinne der Erfindung um eine chemische Bindung wie sie in Römpp Chemie Lexikon, 9. Auflage, Band 1, Seite 650 ff, Georg Thieme Verlag Stuttgart definiert ist, bevorzugt um eine Hauptvalenz Bindung, insbesondere eine kovalente Bindung.

Bei den Substanzen handelt es sich in einer Variante um Arzneimittel oder Wirkstoffe, definiert gemäß Arzneimittelgesetz §2 beziehungsweise. §4 (19), Stand September 2012. In einer Alternative sind Wirkstoffe therapeutisch aktive Stoffe die als arzneilich wirksame Stoffe verwendet werden. Bevorzugt werden Entzündungshemmer eingesetzt.

Bei den Substanzen handelt es sich in einer weiteren Variante um Verbindungen die die Wirkung der Peptide verstärken.

In einer weiteren Alternative handelt es sich um Verbindungen, die die Löslichkeit der Peptide und/oder die Passage der Blut-Hirn-Schranke verbessern.

In einer Alternative haben die Peptide erfindungsgemäß jede beliebige Kombination von mindestens zwei oder mehr Merkmalen der oben beschriebenen Varianten, Ausführungen und/oder Alternativen.

Das erfindungsgemäße Peptid ist ferner vorzugsweise dadurch gekennzeichnet, dass mehrere Peptide der SEQ ID NO: 1, SEQ ID NO: 2, oder SEQ ID NO: 5 kovalent oder nicht kovalent miteinander verknüpft sind.

Eine kovalente Verbindung bzw. Verknüpfung der Peptid-Einheiten liegt im Sinne der Erfindung vor, falls die Peptide Kopf an Kopf, Schwanz an Schwanz oder Kopf an Schwanz linear miteinander verknüpft werden, mit oder ohne dazwischen eingefügte Linker oder Linker-Gruppen.

Das erfindungsgemäße Peptid ist ferner vorzugsweise, dadurch gekennzeichnet, dass mehrere Peptide der SEQ ID NO: 1, SEQ ID NO: 2, oder SEQ ID NO: 5 ohne Linker, also unmittelbar miteinander verknüpft, oder mit einer Linker-Gruppe miteinander verknüpft sind.

Eine nicht kovalente Verknüpfung im Sinne der Erfindung liegt vor, falls die Peptide beispielsweise über Biotin und Streptavidin, insbesondere Streptavidin-Tetramer miteinander verknüpft sind.

Das erfindungsgemäße Peptid ist ferner vorzugsweise, dadurch gekennzeichnet, dass mehrere Peptide der SEQ ID NO: 1, SEQ ID NO: 2 oder SEQ ID NO: 5 linear oder verzweigt miteinander verknüpft sind.

In einer Variante der vorliegenden Erfindung können die Peptide linear miteinander verknüpft sein, insbesondere wie oben beschrieben. In einer anderen Variante sind die Peptide verzweigt miteinander zu dem erfindungsgemäßen Peptid verknüpft.

Bei einem verzweigten Peptid kann es sich erfindungsgemäß um ein Dendrimer handeln, bei dem die Monomere kovalent oder nicht kovalent miteinander verknüpft sind.

Alternativ können die Peptide auch mit einem Plattform-Molekül (wie z. B. PEG oder Zucker) verknüpft sein und so ein verzweigtes Peptid bilden.

Alternativ sind auch Kombinationen dieser Optionen möglich
In einer Ausgestaltung der Erfindung ist die Affinität der Bindung der Peptide über die Dissoziationskonstante (K_{D}-Wert) definiert.

Die Dissoziationskonstante (K_{D}-Wert) eines erfindungsgemäßen Peptids ist dabei in einer vorteilhaften Ausgestaltung der Erfindung vorteilhaft erniedrigt. Damit verbunden sind verbesserte Eigenschaften der erfindungsgemäßen Peptide, wie höhere Affinität der Bindung und höhere Effektivität des Abbaus und/oder der Verhinderung der Bildung toxischer Tau-Oligomere oder Aggregate.

In einer Variante der Erfindung werden solche Peptide eingesetzt, die an ein Tau-Monomer mit einer Dissoziationskonstante (K_{D}-Wert) von höchstens 500 µM, bevorzugt 250, 100, 50 µM, besonders bevorzugt 25, 10, 1 µM, besonders bevorzugt mit einer Dissoziationskonstante (K_{D}-Wert) von höchstens 500 nM, 250, 100, 50, besonders bevorzugt 25, 10, 1 nM, 500 pM, 100, 50, 25, 20, 15, 10, 9, 8, 7, 6, 5, 4, 3, 2, 1 pM bis sub-pM bindet, wobei jeder Zwischenwert angenommen werden kann.

Die erfindungsgemäßen Peptide sind ferner vorzugsweise, dadurch gekennzeichnet, dass sie mit einer K_{D} von kleiner als 1 µM an das Tau-Protein, vorzugsweise das nativ gefaltete Tau-Protein, binden.

Die Peptide können beispielsweise über chemische Synthese bzw. Peptidsynthese hergestellt werden.

In einer weiteren Variante handelt es sich um ein erfindungsgemäßes Peptid zur Hemmung bzw. zur Verhinderung der Bildung von Tau-Peptid-Oligomeren und/oder Tau-Peptidaggregaten.

In einer weiteren Variante handelt es sich um ein erfindungsgemäßes Peptid zur Enttoxifizierung von Tau-Peptid-Oligomeren und/oder Tau-Peptidaggregaten.

Die erfindungsgemäßen Peptide enttoxifizieren die Tau-Peptid-Oligomere und/oder Tau-Peptidaggregate oder daraus gebildete Polymere, sowie Fibrillen, vorzugsweise indem sie nicht daran binden sondern an Tau-Monomer binden und durch Verschiebung des Gleichgewichts zur Reduktion der Tau-Oligomere führen und diese somit in nicht toxische Verbindungen überführen. Demgemäß ist Gegenstand der vorliegenden Erfindung auch ein Verfahren zur Enttoxifizierung der Tau-Oligomeren, daraus gebildeten Aggregaten oder Fibrillen.

Die Hemmung bzw. zur Verhinderung der Bildung von Tau-Peptid-Oligomeren und/oder Tau-Peptid-Aggregaten, bzw. die Enttoxifizierung der Tau-Peptid-Oligomeren und/oder Tau-Peptid-Aggregate kann dabei in vitro oder in vivo erfolgen.

Die vorliegende Erfindung betrifft auch ein Peptid gemäß irgendeinem der vorhergehenden Ansprüche zur Verwendung in der Behandlung von Tauopathien.

Die vorliegende Erfindung betrifft auch ein Peptid gemäß irgendeinem der vorhergehenden Ansprüche insbesondere zur Verwendung in der Behandlung der Alzheimer'schen Demenz (Alzheimer).

Die Erfindung wird nachfolgend in nicht beschränkenden Beispielen näher erläutert.

### Beispiele

Die Peptide TF2D-1, TF2D-5 und TF2D-5a wurden näher untersucht.

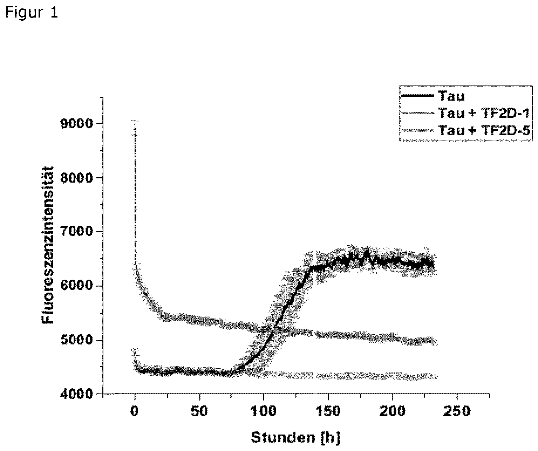

Die Ergebnisse sind in den Figuren 1, 2a, 2b, 3, 4, 5 und 6 zusammengefasst.

### Figur 1:TF2D-1 und TF2D-5 hemmen die Tau-Aggregation, analysiert durch ThT-Analyse

Dargestellt sind die Effekte von TF2D-1 und TF2D-5 auf die Tau-Aggregation. Der ThT-Assay wurde durch Inkubation von 15 µM Tau mit 8 µM Heparin, 20 µM THT und 15 µM eines der beiden Peptide TF2D-1 bzw. TF2D-5 bei 37°C in PBS pH 7,4 durchgeführt. Die Probe ohne Zugabe des Peptids ist rot dargestellt. Die Proben, in denen TF2D-1 oder TF2D-5 vorhanden war, sind grün (TF2D-1) und blau (TF2D-5) dargestellt.

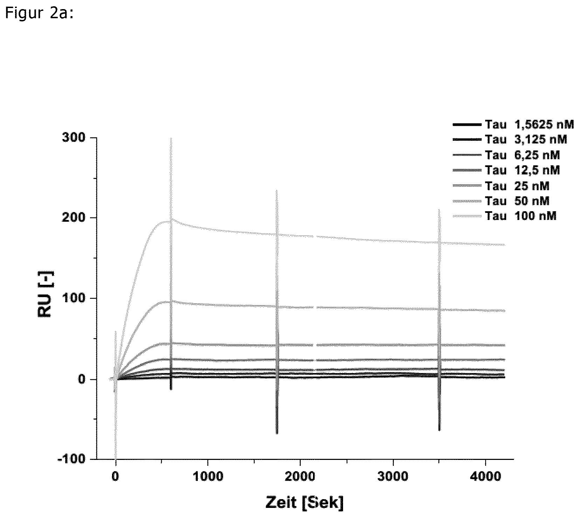

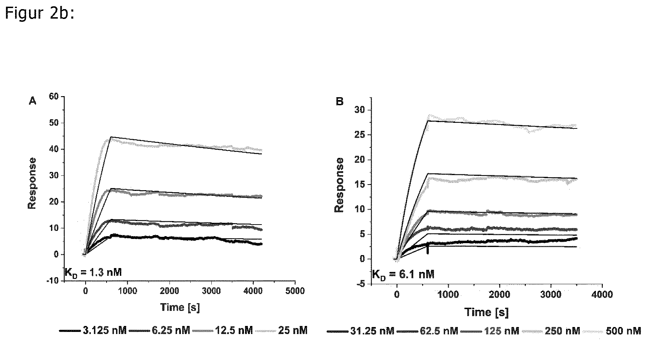

### Figur 2a: TF2D-5 bindet mit hoher Affinität an Tau-Protein

SPR-Messungen von TF2D-5 mit dem Analyten Tau. Für die Messung wurden sieben Tau-Konzentrationen hergestellt, wobei die höchste Konzentration bei 100 nM und die niedrigste Konzentration bei 1,56 nM lag. Der Laufpuffer enthielt TBS mit 0,1% Tween. Der Koff wurde auf etwa 6x10-5 s-1 bestimmt.

### Figur 2b: TF2D-5 und TF2D-5a binden mit hoher Affinität an das Tau-Protein

SPR-Messung von TF2D-5 und TF2D-5a mit volllängen-Tau 441 A) Die Bindungskurven und der berechnete Fit für die Affinität von TF2D-5 zu monomerem Volllängen-Tau 441 ergaben eine K_{D} von 1,3 nM. Die Tau-Konzentration während der Messung variierte von 3,125 nM bis 25 nM. B) Die Bindungsaffinität von TF2D-5a zu Tau in voller Länge wurde mit 6,1 nM bestimmt. Tau wurde in Konzentrationen von 31,25 nM bis 500 nM verwendet. Die SPR-Messungen von A und B wurden auf einem T200 Biacore-Gerät aufgezeichnet, das jeweilige Peptid wurde auf einem CM5-Chip von Cytiva immobilisiert, während monomeres Tau als Analyt diente. Die Tests wurden mit TBS pH 7,4, 0,05 % Tween 20 und 10 µM DTT als Laufpuffer durchgeführt. Die Fit-Berechnung wurde mit der internen Auswertungssoftware des T200 Biacore durchgeführt. Für beide Fits wurde ein 1:1-Modell verwendet.

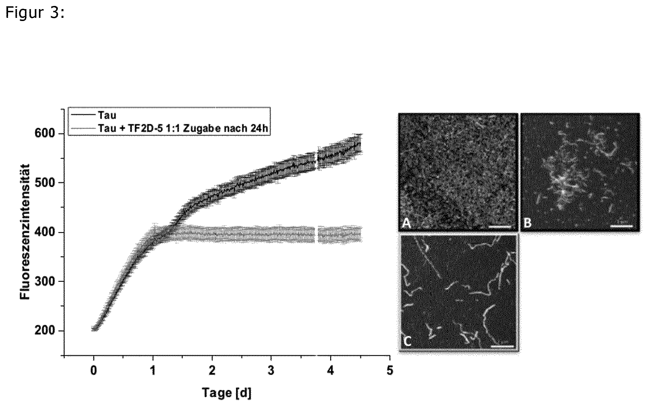

### Figur 3: TF2D-5 hemmt die Tau-Aggregation, analysiert durch ThT- und AFM-Analyse

Dargestellt sind die Auswirkungen von TF2D-5 auf die Tau-Aggregation. Der ThT-Assay wurde durch Inkubation von 15 µM Tau mit 8 µM Heparin, 20 µM ThT, 10% DMSO und unterschiedlichen Konzentrationen von TF2D-5 bei 37°C pH 7,0 durchgeführt. Die Bilder neben der Grafik zeigen die jeweiligen AFM-Messungen der einzelnen Proben, die nach 5 Tagen durchgeführt wurden. In A ist die Probe abgebildet, die kein TF2D-5 enthielt. 6 zeigt die Probe von A mit einer Verdünnung von 1:10. Das mit C markierte Bild entspricht der blauen ThT-Kurve ohne Verdünnung, bei der TF2D-5 nach 24 Stunden zugegeben wurde. Alle AFM-Messungen wurden durch Trocknen von 1 µl auf einer Mica, gefolgt von zwei Waschschritten mit 100 µl Wasser und Messung auf JPK Nanowizard3 mit intermittierender Kontaktluft und einem OMCL AC160TS als Cantilever durchgeführt.

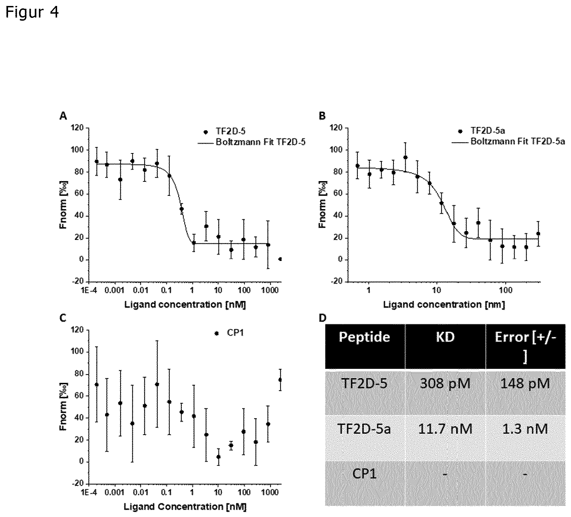

### Figur 4: TF2D-5 und TF2D-5a Affinität an Tau ist sequenzspezifisch

MST-Messung von TF2D-5, TF2D-5a und CP1 mit Tau in voller Länge. A) Boltzmann-Fit von TF2D-5 MST-Messungen mit Tau in voller Länge. TF2D-5 wurde mit CF633 markiert. Die Konzentration von TF2D-5 betrug 500 nM, während die Konzentration von Tau in voller Länge zwischen 2,5 µM und 0,2 pM lag. Die Messung wurde in 1x PBS pH 7,4 mit 150 mM NaCl durchgeführt. Der errechnete K_{D} beträgt 308 pM +/- 148 pM. B) Boltzmann-Fit von TF2D-5a MST-Messungen mit Tau in voller Länge. TF2D-5a wurde mit CF633 markiert. Die Konzentration von TF2D-5a betrug 62,5 nM, während die Konzentration von Tau in voller Länge zwischen 300 nM und 0,5 nM variierte. Die Messung wurde in 0,1 M Natriumphosphatpuffer pH 7,4 mit 75 mM Natriumsulfat und 0,05 % Tween 20 durchgeführt. Der errechnete K_{D} beträgt 11.7 nM +/- 1.3 nM. C) Durchschnitt der CP1-MST-Messungen mit Tau in voller Länge. CP1 besteht aus den gleichen Aminosäuren wie TF2D-5, jedoch mit einer anderen Verteilung der Aminosäuren. CP1 wurde mit CF633 markiert. Die Konzentration von CP1 betrug 62,5 nM, während die Konzentration von Tau in voller Länge zwischen 2,5 µM und 0,2 pM variierte. Die Messung wurde in 0,1 M Natriumphosphatpuffer pH 7,4 mit 75 mM Natriumsulfat und 0,05 % Tween 20 durchgeführt. Es konnte für den getesteten Konzentrationsbereich kein Boltzmann fit und kein K_{D} berechnet werden. D) Berechnete K_{D}s der einzelnen Peptide zu Tau in voller Länge im Überblick. Alle Experimente wurden viermal wiederholt und der Durchschnitt aller Messungen wurde berechnet, der wiederum zur Berechnung der Fit's (Boltzmann) verwendet wurde.

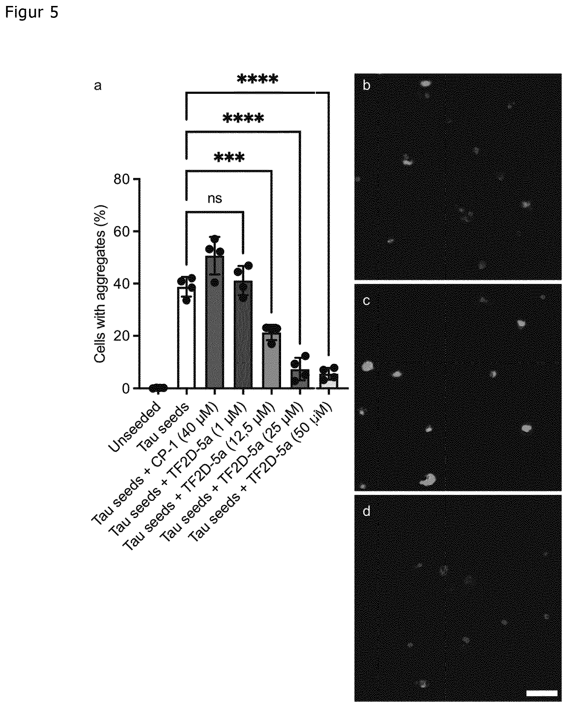

### Figur 5: TF2D-5a hemmt die Aggregation von Tau in Zellen.

A) TF2D-5a und das D-Peptid CP-1, als Negativkontrolle, wurden auf ihre Fähigkeit getestet, die Tau-Aggregation in tauK18(LM)-YFP-Zellen zu hemmen. Der Skalenbalken entspricht 50 µm und gilt für b-d. Die Signifikanz wurde mit Hilfe einer Einweg-ANOVA und dem anschließenden Dunnett-Test für multiple Vergleiche berechnet. Drei Sternchen stehen für einen p-Wert von weniger als 0,001 und vier Sternchen für einen p-Wert von weniger als 0,0001. B) Im Gegensatz zu ungeseedeten Zellen, die am dritten Tag nach der Auspflanzung keine Tau-Aggregate aufwiesen, führte das Seeden mit sonifizierten Tau-Fibrillen nach dreitägiger Inkubation bei etwa 40 % der Zellen zur Aggregation. C) Eine dreitägige Behandlung mit dem D-Peptid CP-1 als Negativkontrolle hemmte die Tau-Aggregation nicht. D) Im Gegensatz dazu führte die Behandlung mit steigenden Konzentrationen von TF2D-5a zu einer konzentrationsabhängigen Verringerung der Anzahl der Zellen mit Tau-Aggregaten, die bei 12,5 µM signifikant war und mit 25 µM und 50 µM TF2D-5a weiter reduziert wurde.

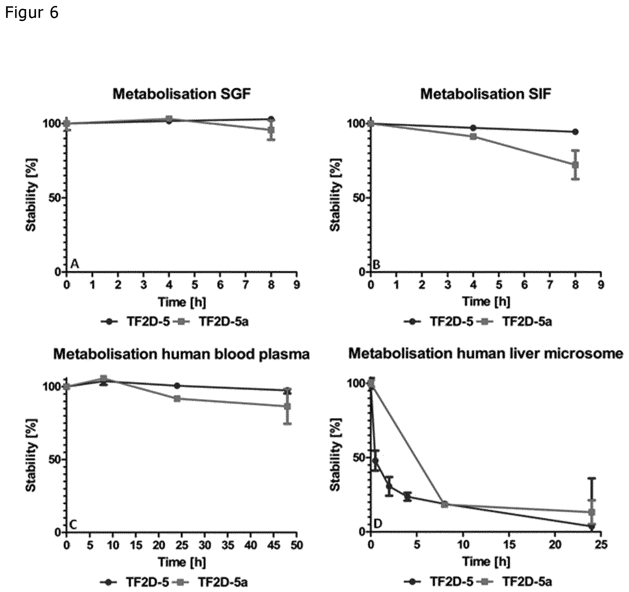

### Figur 6: TF2D-5 und TF2D-5a sind sehr stabil in verschiedenen Körperflüssigkeiten

Stabilitätsuntersuchung von TF2D-5 und TF2D-5a in verschiedenen simulierten menschlichen Körperflüssigkeiten. A) Stabilität der Peptide in simulierter Magenflüssigkeit (SGF). Die simulierte Magenflüssigkeit bestand aus 2 mg/ml Natriumchlorid, 3,2 mg/ml Pepsin und 80 mM HCl; pH 1. B) Stabilität von TF2D-5 und TF2D-5a in simulierter Darmflüssigkeit (SIF). Simulierte Darmflüssigkeit bestand aus 6,8 mg/ml Kaliumdihydrogenphosphat, 10 mg/ml Pankreaspulver und 15,4 mM NaOH; pH 6,8. C) Stabilität von TF2D 5 und TF2D-5a in menschlichem Blutplasma. Humanes K3EDTA-Blutplasma wurde von BioTrend bezogen. D) Stabilität von TF2D-5 und TF2D-5a in menschlichen Lebermikrosomen. Menschliche Lebermikrosomen bestanden aus 6 mg/ml Protein (das Cytochrom P450 (CYP)-Enzyme enthält), 75 mM Saccharose, 1,3 mM NADPH, 3,3 mM D-Glucose-6-phosphat, 0,2 u/ml D-Glucose-6-phosphat-Dehydrogenase, 3,3 mM Magnesiumchlorid und 100 mM Kaliumphosphat pH 7,4.

## Patentansprüche

1. Peptid, bestehend aus einer Aminosäuresequenz ausgewählt aus der Gruppe bestehend aus SEQ ID NO: 1, SEQ ID NO: 2, und SEQ ID NO: 5, **dadurch gekennzeichnet, dass** das Peptid aus D-Aminosäuren besteht.

2. Peptid gemäß Anspruch 1, **dadurch gekennzeichnet, dass** am freien C-terminus an Stelle der Carboxylgruppe eine Säureamidgruppe (CONH₂-Gruppe) oder eine COH-Gruppe, COCI-Gruppe, COBr-Gruppe, CONH-alkyl-Rest oder ein CONH-alkyl-Amin-Rest vorliegt oder aber das Peptid zyklisiert vorliegt.

3. Peptid gemäß Anspruch 1 oder Anspruch 2, **dadurch gekennzeichnet, dass** es 2, 3, 4, 5, 6, 7, 8, 9, 10 oder mehr Kopien der Sequenzen mit der SEQ ID NO: 1, SEQ ID NO: 2 oder SEQ ID NO: 5 enthält.

4. Peptid gemäß irgendeinem der vorhergehenden Ansprüche, **dadurch gekennzeichnet, dass** das Peptid mit einer weiteren Substanz verknüpft ist.

5. Peptid gemäß irgendeinem der vorhergehenden Ansprüche, **dadurch gekennzeichnet, dass** mehrere Peptide der SEQ ID NO: 1, SEQ ID NO: 2, oder SEQ ID NO: 5 kovalent oder nicht kovalent miteinander verknüpft sind.

6. Peptid gemäß irgendeinem der vorhergehenden Ansprüche, **dadurch gekennzeichnet, dass** mehrere Peptide der SEQ ID NO: 1, SEQ ID NO: 2, oder SEQ ID NO: 5 ohne Linker, also unmittelbar miteinander verknüpft, oder mit einer Linker-Gruppe miteinander verknüpft sind.

7. Peptid gemäß irgendeinem der vorhergehenden Ansprüche, **dadurch gekennzeichnet, dass** mehrere Peptide der SEQ ID NO: 1, SEQ ID NO: 2, oder SEQ ID NO: 5 linear oder verzweigt miteinander verknüpft sind.

8. Peptid gemäß irgendeinem der vorhergehenden Ansprüche, **dadurch gekennzeichnet, dass** es sich um ein Dendrimer handelt, wobei Peptide der SEQ ID NO: 1, SEQ ID NO: 2, oder SEQ ID NO: 5 mit einem Plattform-Molekül verknüpft sind.

9. Peptid gemäß irgendeinem der vorhergehenden Ansprüche, **dadurch gekennzeichnet, dass** es mit einer K_{D} von kleiner als 1µM an das Tau-Peptid bindet.

10. Peptid gemäß irgendeinem der vorhergehenden Ansprüche zur Verwendung in einem Verfahren zur Verhinderung der Bildung von Tau-Peptid-Oligomeren und/oder Tau-Peptid-Aggregaten.

11. Peptid gemäß irgendeinem der vorhergehenden Ansprüche zur Verwendung in einem Verfahren zur Enttoxifizierung von Tau-Peptid-Oligomeren und/oder Tau-Peptid-Aggregaten.

12. Peptid gemäß irgendeinem der vorhergehenden Ansprüche zur Verwendung in einem Verfahren zur Behandlung von Tauopathien.

## Claims

1. A peptide consisting of an amino acid sequence selected from the group consisting of SEQ ID NO: 1, SEQ ID NO: 2, and SEQ ID NO: 5, **characterized in that** the peptide consists of D-amino acids.

2. The peptide according to claim 1, **characterized in that** an acid amide group (CONH₂ group) or a COH group, COCI group, COBr group, CONH alkyl residue, or a CONH alkyl amine residue is present at the free C-terminus in place of the carboxyl group, or the peptide is present in a cyclized form.

3. The peptide according to claim 1 or claim 2, **characterized in that** it contains 2, 3, 4, 5, 6, 7, 8, 9, 10 or more copies of the sequences of SEQ ID NO: 1, SEQ ID NO: 2 or SEQ ID NO: 5.

4. The peptide according to any one of the preceding claims, **characterized in that** the peptide is linked to a further substance.

5. The peptide according to any one of the preceding claims, **characterized in that** several peptides of SEQ ID NO: 1, SEQ ID NO: 2, or SEQ ID NO: 5 are covalently or non-covalently linked to each other.

6. The peptide according to any one of the preceding claims, **characterized in that** several peptides of SEQ ID NO: 1, SEQ ID NO: 2, or SEQ ID NO: 5 are linked without a linker, i.e., directly to each other, or are linked to each other with a linker group.

7. The peptide according to any one of the preceding claims, **characterized in that** several peptides of SEQ ID NO: 1, SEQ ID NO: 2, or SEQ ID NO: 5 are linked to each other in a linear or branched manner.

8. The peptide according to any one of the preceding claims, **characterized in that** it is a dendrimer, wherein peptides of SEQ ID NO: 1, SEQ ID NO: 2, or SEQ ID NO: 5 are linked to a platform molecule.

9. The peptide according to any one of the preceding claims, **characterized in that** it binds to the tau peptide with a K_{D} of less than 1 µM.

10. The peptide according to any one of the preceding claims for use in a method for preventing the formation of tau peptide oligomers and/or tau peptide aggregates.

11. The peptide according to any one of the preceding claims for use in a method for detoxifying tau peptide oligomers and/or tau peptide aggregates.

12. The peptide according to any one of the preceding claims for use in a method for treating tauopathies.

## Revendications

1. Peptide constitué d'une séquence d'acides aminés sélectionnée dans le groupe constitué par les séquences SEQ ID NO: 1, SEQ ID NO: 2 et SEQ ID NO: 5, **caractérisé en ce que** le peptide est constitué d'acides aminés D.

2. Peptide selon la revendication 1, **caractérisé en ce que**, à la terminaison C libre, au lieu du groupe carboxyle, un groupe amide (groupe CONH₂) ou un groupe COH, un groupe COCl, un groupe COBr, un résidu alkyle CONH ou un résidu alkylamine CONH est présent, ou **en ce que** le peptide est cyclisé.

3. Peptide selon la revendication 1 ou la revendication 2, **caractérisé en ce qu'**il contient 2, 3, 4, 5, 6, 7, 8, 9 ou 10 copies ou plus des séquences SEQ ID NO: 1, SEQ ID NO: 2 ou SEQ ID NO: 5.

4. Peptide selon l'une quelconque des revendications précédentes, **caractérisé en ce que** le peptide est lié à une autre substance.

5. Peptide selon l'une quelconque des revendications précédentes, **caractérisé en ce que** plusieurs peptides de la séquence SEQ ID NO: 1, SEQ ID NO: 2 ou SEQ ID NO: 5 sont liés entre eux par des liaisons covalentes ou non covalentes.

6. Peptide selon l'une quelconque des revendications précédentes, **caractérisé en ce que** plusieurs peptides de la séquence SEQ ID NO: 1, SEQ ID NO: 2 ou SEQ ID NO: 5 sont liés entre eux sans lieur, c'est-à-dire directement, ou par un groupe lieur.

7. Peptide selon l'une quelconque des revendications précédentes, **caractérisé en ce que** plusieurs peptides de la séquence SEQ ID NO: 1, SEQ ID NO: 2 ou SEQ ID NO: 5 sont liés ensemble de manière linéaire ou ramifiée.

8. Peptide selon l'une quelconque des revendications précédentes, **caractérisé en ce qu'**il s'agit d'un dendrimère, dans lequel des peptides selon la séquence SEQ ID NO: 1, SEQ ID NO: 2 ou SEQ ID NO: 5 sont liés par une molécule de plateforme.

9. Peptide selon l'une quelconque des revendications précédentes, **caractérisé en ce qu'**il se lie au peptide tau avec un K_{D} inférieur à 1 µM.

10. Peptide selon l'une quelconque des revendications précédentes, destiné à être utilisé dans un procédé de prévention de la formation d'oligomères du peptide tau et/ou d'agrégats du peptide tau.

11. Peptide selon l'une quelconque des revendications précédentes, destiné à être utilisé dans un procédé de détoxification d'oligomères du peptide tau et/ou d'agrégats du peptide tau.

12. Peptide selon l'une quelconque des revendications précédentes, destiné à être utilisé dans un procédé de traitement des tauopathies.
